# EUROPEAN PATENT SPECIFICATION

(11) **EP 3 752 004 B1**
(45) Date of publication and mention of the grant of the patent: **03.07.2024**
(21) Application number: 19704634.5
(22) Date of filing: 18.02.2019
(51) Int. Cl.: A23J 1/00, A23J 1/12, A23K 10/38, A23J 3/14, A23K 10/12, A23K 10/14, A23L 5/20, A23L 33/185

(54) **PROCESS FOR RECOVERING PROTEINACEOUS AND FIBROUS MATERIAL FROM BREWERS' SPENT GRAINS, AND USE THEREOF**
VERFAHREN ZUR GEWINNUNG VON PROTEIN- UND FASERMATERIAL AUS BRAUEREITREBER VON UND SEINE VERWENDUNG
PROCÉDÉ DE RÉCUPÉRATION DE MATIÈRES PROTÉINÉES ET FIBREUSES À PARTIR DE GRAINES DE BRASSERIE ET SON UTILISATION

(30) Priority: 16.02.2018 BE 201805096; 21.02.2018 EP 18157847
(43) Date of publication of application: 23.12.2020
(73) Proprietor: Anheuser-Busch InBev S.A., 1000 Brussels (BE)
(72) Inventor: GIL-MARTINEZ, Jorge, 3000 Leuven (BE); ARENDT, Elke, Cork City (IE)
(74) Representative: Marks & Clerk LLP
(86) International application number: PCT/EP2019/053977
(87) International publication number: WO 2019/158755

(56) References cited:
- GB-A- 2 220 124
- INGE CELUS ET AL: "Enzymatic Hydrolysis of Brewers' Spent Grain Proteins and Technofunctional Properties of the Resulting Hydrolysates", JOURNAL OF AGRICULTURAL AND FOOD CHEMISTRY, vol. 55, no. 21, 1 October 2007 (2007-10-01), pages 8703-8710, XP055005691, ISSN: 0021-8561, DOI: 10.1021/jf071793c
- INGE CELUS ET AL: "Fractionation and Characterization of Brewers' Spent Grain Protein Hydrolysates", JOURNAL OF AGRICULTURAL AND FOOD CHEMISTRY, vol. 57, no. 12, 24 June 2009 (2009-06-24) , pages 5563-5570, XP055511464, US ISSN: 0021-8561, DOI: 10.1021/jf900626j
- JANNEKE TREIMO ET AL: "Enzymatic Solubilization of Brewers' Spent Grain by Combined Action of Carbohydrases and Peptidases", JOURNAL OF AGRICULTURAL AND FOOD CHEMISTRY, vol. 57, no. 8, 22 April 2009 (2009-04-22) , pages 3316-3324, XP055511480, US ISSN: 0021-8561, DOI: 10.1021/jf803310f
- PIRKKO FORSSELL ET AL: "Hydrolysis of Brewers' Spent Grain by Carbohydrate Degrading Enzymes", JOURNAL OF THE INSTITUTE OF BREWING., vol. 114, no. 4, 1 January 2008 (2008-01-01), pages 306-314, XP055511502, GB ISSN: 0046-9750, DOI: 10.1002/j.2050-0416.2008.tb00774.x
- SOLANGE I MUSSATTO ET AL: "Brewer's spent grain as raw material for lactic acid production by Lactobacillus delbrueckii", BIOTECHNOLOGY LETTERS, SPRINGER NETHERLANDS, DORDRECHT, vol. 29, no. 12, 14 August 2007 (2007-08-14), pages 1973-1976, XP019548734, ISSN: 1573-6776, DOI: 10.1007/S10529-007-9494-3

## Description

### Field of the Invention

The present invention concerns a process of extracting or purifying proteinaceous material and fibraceous material from brewer's spent grain, as well as the use of the extracted/purified proteinaceous and fibraceous material obtained from the brewer's spent grain.

### Background to the Invention

Brewers' spent grain (BSG) is the most abundant co-product generated in the beer-brewing process. This material consists of the barley grain husks obtained as solid portion after the wort production. Since BSG is rich in sugars and proteins, the main use to date for the utilization of this product has been as animal feed. However, for exactly these same reasons, because it is high in dietary fiber and proteins, BSG is of interest for application in different areas particularly when considering its valuable component composition as a potential source of bioactive, health-promoting compounds.

BSG consists of the seed coat-peri carp-husk layers that covered the original barley grain. The starch content is usually low, and the composition of BSG mainly contains fibers, which are non-starch polysaccharides (NSP; hemicellulose in the form of arabinoxylans (AX) and cellulose) and significant quantities of proteins and lignin, with arabinoxylans (AX) typically constituting the most abundant component. Therefore, BSG is basically a lignocellulosic material. Fiber constitutes about half of the BSG composition on a dry weight basis, while proteins can constitute up to 30% of the dry weight basis. This high fiber and protein content makes BSG an interesting raw material for food applications.

As would be expected, cellulose (β-(1,4)-linked glucose residues) is another abundant polysaccharide in BSG. Certain levels of (1-3,1-4)-β-D-glucan may also be present. The most abundant monosaccharides in BSG are xylose, glucose, and arabinose, while traces of traces of rhamnose and galactose have also been found.

Arabinoxylans (AX) constitute up to 25% of dry weight in BSG. Most of these are associated with other fibre components (cellulose or lignin) or with protein and are not bioavailable (water-unextractable arabinoxylans, WUAX). A small fraction of WUAX can be made soluble (water-extractable arabinoxylans, WEAX) via enzymatic treatment Consumption of WEAX has been shown to have positive health effects, including prebiotic effects, regulation of postprandial blood glucose levels, lowering cholesterol levels, tumor suppression and immunomodulating effects. It is, therefore, desirable to increase the proportion of WEAX in BSG preparations for human consumption. The protein content of BSG typically is present at levels of approximately 30% per dry weight basis. The most abundant are hordeins, glutelins, globulins and albumins.

Essential amino acids represent approximately 30% of the total protein content, with lysine being the most abundant, while non-essential amino acids in BSG constitute up to 70% of the total protein content. This is significant because lysine is often deficient in cereal foods. In addition, BSG also contains a variety of minerals elements, among which silicon, phosphorus, calcium and magnesium are the most abundant.

Celus et al, Enzymatic hydrolysis of brewers' spent grain proteins and technofunctional properties of the resulting hydrolysates, J. Agri. Food Chem., 2007, 55, 8703-8710 discloses a process for preparing a BSG hydrolysate by alkaline extraction of BSG and acid precipitation to prepare a BSG protein concentrate, which was hydrolysed using commercial proteases. Celus et al, Fractionation and characterization of brewers' spent grain protein hydrolysates, J. Agri. Food Chem., 2009, 57, 5563-5570 discloses a process for preparing a BSG hydrolysate by alkaline extraction of BSG and acid precipitation to prepare a BSG protein concentrate, which was hydrolysed using Alcalase and then fractionated by precipitation. Treimo et al, Enzymatic solubilization of brewers' spent grain by combined action of carbohydrases and peptidases, J. Agri. Food Chem., 2009, 57, 3316-3324 discloses a process for solubilizing BSG by the combined action of carbohydrases and peptidase. GB2220124A discloses a process for the preparation of animal feed comprising the treatment of spent grains with one or more carbohydrate lyase enzymes, said grains containing or being subsequently contacted with lactic acid producing microorganisms.

The present invention is directed in particular to the recovery of proteinaceous and fibraceous material from BSG by first altering the composition of freshly collected BSG to either improve the recovery yield of proteinceous and fibracious material or by increasing the functional value of the recovered proteinaceous and fibracious material, thereby allowing obtaining a proteinaceous and fibraceous material with a potentially beneficial effect on the organization of the intestinal microbial community when used as a supplement in food or feed as a source of protein and/or an increased level of health-promoting WEAX. As such, the present invention does not only address new uses of brewer's spent grain, but specifically addresses a higher valorization of the brewer's spent grain than currently possible.

### Summary of the Invention

The present invention achieves a high valorization of brewer's spent grain by use of this material as a source for recovering proteinaceous and fibraceous material therefrom. The recovered protein can be applied in numerous applications such as beverages with a high protein content desired by sportsmen and craftsmen to recover from intense physical exercise. The fibraceous material can be used for supplementing e.g. food or feed, to increase fiber content, a proportion of which fibres is comprised by health-promoting water-extractable arabinoxylans (WEAX).

In particular, the present invention concerns a process for recovering proteinaceous and fibraceous material from brewer's spent grains, the method comprising the steps of:
a) Providing brewer's spent grain (BSG);
b) Performing enzymatic treatment of the brewer's spent grain, the enzymatic treatment of the brewer's spent grain including addition of xylanase, to form an enzymatically treated brewer's spent grain;
c) Fermenting the enzymatically treated brewer's spent grain with lactic acid bacteria and/or acetic acid bacteria and/or probiotics to obtain a fermented broth;
d) Hydrolysing proteins in the fermented broth to provide a hydrolysed fermented broth, wherein the pH of the fermentation broth is adjusted to a pH in the range of 2.5 to 3.5 for the step of hydrolyzing; and,
e) Extracting and/or purifying proteinaceous and fibraceous material from the hydrolysed fermented broth.

The present invention further concerns the use the recovered proteinaceous and material as defined supra as a supplement for food or feed.

The present invention finally concerns the use of lactic acid bacteria (LAB) for improving the recovery yield of proteinaceous or fibraceous material from BSG.

### Detailed Summary of the Invention

The enzyme treatment of the brewer's spent grain includes the addition of xylanase.

Treatment with said enzyme results in an increase of the levels of health-promoting soluble arabinoxylans (WEAX).

Preferably, the fermentation of the fermentable broth is achieved by lactic acid bacteria, preferably lactic acid bacteria of the species *Lactobacillus plantarum* and/or *Lactobacillus rhamnosus,* more preferably the strain *Lactobacillus plantarum* F10 and/or *Lactobacillus rhamnosus* GG (LGG^{®}).

### Definitions

Barley is the main raw material used for the production of beer. However, other cereals such as corn or rice are typically used together with malted barley. During the brewing process the starchy endosperm of these cereals is subjected to enzymatic degradation, resulting in the liberation of fermentable (maltose and maltotriose, and a minor percentage of glucose) and non-fermentable carbohydrates (dextrins), proteins, polypeptides and amino acids. The thus produced medium (which will be fermented into beer by the action of yeast) is known as wort. The insoluble grain components (comprising mainly the grain coverings) is the brewers' spent grain (BSG). In traditional brewing employing a lauter tun, the BSG components play an important role as they form the bed through which the mash is filtered to produce wort. Therefore, the initial milling of the malt must be such that the grain coverings remain intact so as to form an adequate filter. Today, while many small or craft breweries still use this method of mash filtration, many larger breweries employ a mash filter which relies less on the filtration function of the BSG and thus malt can be milled more extensively.

The brewer's spent grain contains all the solids that have been separated from the wort by filtration; it includes what is left of the barley malt and the adjuncts. The spent grain consists mainly of the pericarp and hull portions of the barley and of non-starchy parts of corn, provided corn grits were used as an adjunct. Brewer's spent grain is a lignocellulosic material typically comprising lipids, lignin, proteins, cellulose, hemicellulose and some ash.. For the description and claims of this invention the wording *"brewer's spent grain"* (BSG) will be used in accordance with the definition here above.

Product water refers to water used in the brewing process, that has suffered a defined and standard process for making it suitable for consumption.

Nutritional definitions as defined by the European Commission (http://ec.europa.eu/food/safety/labelling_nutrition/claims/nutrition_claims/index_en.htm) , see Table below:

| **Nutritional claim** | **Definition** |
|---|---|
| ***Low energy*** | <20 kCal per 100 g |
| ***Fat free*** | <0.5% fat content |

| | |
|---|---|
| ***Low fat*** | <1.5% fat content |
| ***Very low salt*** | <0.4% salt content |
| ***Source of fiber*** | >3% fiber content OR >1.5 g fiber per 100 kCal |
| ***Low sugar*** | <2.5% sugar content |
| ***High in fiber*** | >6% fiber content OR >3 g fiber per 100 kCal |
| ***Source of protein*** | >12% of the energy provided by protein |
| ***High in protein*** | >20% of the energy provided by protein |

Digestion of AX either enzymatically or otherwise results in an increase of the soluble fraction of arabinoxylans (WEAX). This fraction is responsible for most of the health-promoting effects of arabinoxylans. Among the many positive effects WEAX have on health we find:
1. reduction of postprandial glucose levels in individuals with compromised glucose metabolism (Lu et al., 2004, Garcia et al., 2006)
2. tumor suppressing activity (Li et al., 2011)
3. reduction of obesity, cholesterol levels and restoration of beneficial gut bacteria in high fat diets (Neyrinck et al., 2011)
4. immune-enhancing effects (Zhou et al., 2010)
5. prebiotic effects, including promoting healthy gut bacteria and short chain fatty acid in distal colon (Cloetens et al., 2010, Sanchez et al., 2009)

Additionally, there is evidence that preparations of arabinoxylans from brewer's spent grains (BSG-AX) can exert the same prebiotic effects as the better-studied wheat-derived arabinoxylans, namely:
6. BSG-AX are not absorbed in the small intestine and reach the colon (Texeira et al., 2017); BSG-AX promote proliferation of gut bacteria, particularly beneficial species like, for example, those of the Bifidobacteria genus, and BSG-AX promote the production of short chain fatty acids by said bacteria (Reis et al., 2014)

The documented effects listed above were elicited by the following dosages:
(1) 0.12 g/kg body weight/day, (2) 0.4 g/kg body weight/day, (3)10% of diet, (4) 0.1 g/kg day , (5) 0.14 g/kg weight/day and 0.6% (w/v), (6) 0.6 g/kg body weight/day

Additionally, a patent concerning the use of soluble arabinoxylans extracted from wheat (Ekhart et al., 2016), recommends that a daily dosage of 0.08 g/kg day would be adequate to obtained the claimed health effects, namely prebiotic effect and decrease of symptoms associated with high-fat diets.

European Food Safety Authority has concluded that there is a cause effect relationship between the consumption of wheat arabinoxylan and the reduction of postprandial glucose levels (EFSA, 2011) . Based on the provided evidence EFSA suggests that to obtain the claimed effect, 4.8 % w/w of consumed carbohydrate should be soluble arabinoxylans. For a healthy 70kg adult with an average 2200 kcal daily intake (EFSA, 2013), of which 45% are carbohydrates (EFSA, 2010), this corresponds to 0.17 g/kg body weight/day.

It is therefore considered that no less than 0.1 g/kg body weight/day, is a sufficient dose of WEAX to have positive health effects.

The fibre-solubilization and saccharification enzyme process described here results in allowing recovery of fibraceous material from the BSG, wherein the fibraceous material comprises no less than 1.4 % (w/v) soluble arabinoxylans.

Finally, lactose free refers to a product that contains no trace of this compound. The present invention refers to a proteinaceous material or fibrous material recovered from BSG through the fermentation of BSGs, therefore containing no dairy product and thus lactose free.

### Detailed Description of a Preferred Embodiment

The process according to the present invention generally comprises the steps of:
a) Providing brewer's spent grain;
b) Performing enzymatic treatment of the brewer's spent grain, the enzymatic treatment of the brewer's spent grain including addition of xylanase, to form an enzymatically treated brewer's spent grain;
c) Fermenting the enzymatically treated brewer's spent grain with lactic acid bacteria and/or acetic acid bacteria and/or probiotics to obtain a fermented broth;
d) Hydrolysing proteins in the fermented broth to provide a hydrolysed fermented broth, wherein the pH of the fermentation broth is adjusted to a pH in the range of 2.5 to 3.5 for the step of hydrolyzing; and,
e) Extracting and/or purifying proteinaceous and/or fibraceous material from the hydrolysed fermented broth.

The brewer's spent grain is preferably obtained from a regular beer production process, wherein malt and potentially some adjuncts such as corn, rice, sorghum, wheat, barley, rye, oat or combinations thereof are mixed with water to form a mash wherein enzymes either originating from the barley malt or added separately to the mash - are allowed to break down starch into fermentable sugars, typically a mixture of glucose, maltose and maltotriose. At the end of the mashing, the mash is filtered to obtain a fermentable wort that is further processed in to beer. The retentate of the mash filtering is the brewer's spent grain (BSG).

BSG comprises the seed coat-pericarp-husk layers that covered the original barley grain. BSG's composition mainly comprises fibers, which are non-starch polysaccharides (NSP; hemicellulose in the form of arabinoxylans (AX) and cellulose) and significant quantities of proteins and lignin, with arabinoxylans (AX) typically constituting the most abundant component. Therefore, BSG is basically a lignocellulosic material. Fiber constitutes about half of the BSG composition on a dry weight basis, while proteins can constitute up to 30% of the dry weight basis. This high fiber and protein content makes BSG an interesting raw material for food applications.

As would be expected, cellulose (β-(1,4)-linked glucose residues) is another abundant polysaccharide in BSG. Certain levels of (1-3,1-4)-β-D-glucan may also be present. The most abundant monosaccharides in BSG are xylose, glucose, and arabinose, while traces of traces of rhamnose and galactose have also been found.

The protein content of BSG typically is present at levels of approximately 30% per dry weight basis. The most abundant are hordeins, glutelins, globulins and albumins. Essential amino acids represent approximately 30% of the total protein content, with lysine being the most abundant, while non-essential amino acids in BSG constitute up to 70% of the total protein content. This is significant because lysine is often deficient in cereal foods. In addition, BSG also contains a variety of minerals elements, among which silicon, phosphorus, calcium and magnesium are the most abundant.

The BSG obtained from a lager beer production process typically comprises hemicellulose (20-25w% on dry matter); cellulose (12-25w% on dry matter); protein (19-30w% on dry matter); lignin (12-28w% on dry matter); lipid (ca. 10w% on dry matter); ash (2-5w% on dry matter); and low amounts of fructose, lactose, glucose and maltose. The BSG is highly nutritious and very sensitive for spoilage by micro-organisms, hence heat treating of the BSG is desired to increase the shelf life. In this sense, the high water content of BSGs in the moment of their production (wort filtration), which is in the range of 75% (25% total solids), increases the instability of the material. For this reasons preferably fresh spent grains are used in the process of the present invention, and/or BSGs are stabilized or treated for sterilization, preferably by boiling.

In a process according to the present invention, BSGs, preferably as produced during the brewing process (in the range of 25% total solid content), and more preferably collected just after their production, are mixed with distilled water, or preferably hot product water, to a final dry matter content of between 6 and 10%, more preferably between 8 and 9%. The solids in this suspension are ground, preferably using corundum stone grinding technology, to an average particle size no bigger than 80 µm and an absolute particle size no bigger than 300 µm. The ground suspension is subsequently treated for stabilization, for example by heat treatment such as by boiling for 60 minutes.

Subsequently, the mixture of BSGs and water is exposed to fibre solubilization, saccharification and fermentation, preferably to a simultaneous process of saccharification and fermentation (SSF). Commercial enzymatic products used for the fibre solubilization and saccharification of the BSG in the present invention will have at least the following activity: xylanase (including endo-xylanase). Preferably, the enzymatic mixture use will contain starch, dextrin, protein and fiber degrading activities. More preferably, these activities will comprise gluco-amylase, pullulanase, alpha-amylase, beta-glucanase, xylanase and protease. Enzyme treatment with xylanase and protease solubilizes WUAX and increases the levels of health-promoting WEAX.

As examples of such enzyme treatment, experiments were done by adding to a mixture of BSGs and water the following commercial products:

### Example 1

| **Commercial Product** | **Supplier** | **Declared enzymatic activities** | **Dose** |
|---|---|---|---|
| ***Ultraflo FABI*** | ***Novozymes*** | Beta-glucanase | 100 ppm |
| | | Endo-xylanase | |
| | | Alpha-amylase | |
| ***Attenuzyme PRO*** | ***Novozymes*** | Gluco-amylase | 500 ppm |
| | | Pullulanase | |
| | | Alpha-amylase | |
| ***Flavourzyme*** | ***Novozymes*** | Protease | 200 ppm |

### Example 2

| **Commercial Product** | **Supplier** | **Declared enzymatic activities** | **Dose** |
|---|---|---|---|
| ***Ultraflo FABI*** | ***Novozymes*** | Beta-glucanase | 100 ppm |
| | | Endo-xylanase | |
| | | Alpha-amylase | |
| ***Attenuzyme PRO*** | ***Novozymes*** | Gluco-amylase | 500 ppm |
| | | Pullulanase | |
| | | Alpha-amylase | |
| ***Food Pro PHT*** | ***DuPont*** | Protease | 100 ppm |
| ***Flavourzyme*** | ***Novozymes*** | Protease | 200 ppm |

### Example 3

| **Commercial Product** | **Supplier** | **Declared enzymatic activies** | **Dose** |
|---|---|---|---|
| ***Laminex BG2*** | ***Danisco*** | Beta-glucanase | 100 ppm |
| | | Xylanase | |
| ***Ultimase BWL40*** | ***Novozymes*** | Beta-glucanase | 800 ppm |
| | | Xylanase | |

### Example 4

| **Commercial Product** | **Supplier** | **Declared enzymatic activities** | **Dose** |
|---|---|---|---|
| ***Allzyme*** | ***Alltech*** | Beta-glucanase | 800 ppm |
| | | Endo-xylanase | |
| | | Cellulase | |
| ***Attenuzyme PRO*** | ***Novozymes*** | Gluco-amylase | 500 ppm |
| | | Pullulanase | |
| | | Alpha-amylase | |
| ***Food Pro PHT*** | ***DuPont*** | Protease | 100 ppm |
| ***Flavourzyme*** | ***Novozymes*** | Protease | 200 ppm |

### Example 5

| **Commercial Product** | **Supplier** | **Declared enzymatic activities** | **Dose** |
|---|---|---|---|
| ***Rohament CL*** | ***AB-Enzymes*** | Beta-glucanase | 800 ppm |
| | | Endo-xylanase | |
| | | Cellulase | |
| ***Attenuzyme PRO*** | ***Novozymes*** | Gluco-amylase | 500 ppm |
| | | Pullulanase | |
| | | Alpha-amylase | |
| ***Food Pro PHT*** | ***DuPont*** | Protease | 100 ppm |
| ***Flavourzyme*** | ***Novozymes*** | Protease | 200 ppm |

After hydrolysis, a fermentable broth is obtained that is subsequently fermented with lactic acid bacteria and/or acetic acid bacteria and/or probiotics. Preferably, such microorganisms are added during the hydrolysis, thus performing a simultaneous saccharification and fermentation process (SSF). The lactic acid bacteria can be used either alone or in combination with yeast (eg *S. cerevisiae*)*.*

Examples of lactic acid bacteria include:

| **Species** | **Strain** | **Metabolism** | **Origin** |
|---|---|---|---|
| *L. amylovorus* | AB32 | Homofermentative | Sourdough |
| *L. amylovorus* | AB36 | Homofermentative | Sourdough |
| *L. brevis* | WLP672 | Heterofermentative | |
| *L. brevis* | JJ2P | Heterofermentative | Porcine |
| *L. paracasei* | CRL431 | Heterofermentative | Infant faeces |
| *L. casei* | R10 | Heterofermentative | Cheese |
| *L. casei* | H2 | Heterofermentative | Human |
| *L. crispaticus* | AB19 | Homofermentative | Sourdough |
| *L. delbreuckii* | WLP677 | Homofermentative | |
| *L. fermentum* | AB15 | Heterofermentative | Sourdough |
| *L. fermentum* | AB31 | Heterofermentative | Sourdough |
| *L. fermentum* | F23 | Heterofermentative | Sourdough |
| *L. gallinarum* | AB13 | Homofermentative | Sourdough |
| *L. plantarum* | F6 | Heterofermentative | Sourdough |
| *L. plantarum* | F10 | Heterofermentative | Brewery |
| *L. plantarum* | F21 | Heterofermentative | Sourdough |
| *L. plantarum* | R11 | Heterofermentative | Cheese |
| *L. plantarum* | R13 | Heterofermentative | Cheese |
| *L. reuteri* | AB38 | Heterofermentative | Sourdough |
| *L. reuteri* | DSM20016 | Heterofermentative | Human intestine |
| *L. reuteri* | Ff2 | Heterofermentative | Porcine |
| *L. reuteri* | hh1P | Heterofermentative | Porcine |
| *L. reuteri* | R12 | Heterofermentative | Cheese |
| *L. rhamnosus* | C7 | Homofermentative | Cheese |
| *L. rhamnosus* | C8 | Homofermentative | Cheese |
| *L. rhamnosus* | C9 | Homofermentative | Cheese |
| *L. rhamnosus* | GG | Homofermentative | Human gut |
| *L. sakei* | AB3a | Heterofermentative | Sourdough |
| *L. vaginalis* | AB11 | Heterofermentative | Sourdough |
| *Leuconostoc citreum* | TR116 | Heterofermentative | Sourdough |
| *L. holzapfelii* | AB4 | Heterofermentative | Sourdough |
| *Leuconostoc lactis* | E11 | Heterofermentative | Sourdough |
| *Leuc. Mesenteroides* | DSM20240 | Heterofermentative | Root beer |
| *Weissella cibaria* | MG1 | Heterofermentative | Sourdough |

Examples of acetic acid bacteria include *G*. *oxydans* and *K. xylinus.*

Preferably, the strains *L. planetarum F10* and *L. rhamnosus LGG* are preferred as selected to provide desirable organoleptic properties to the fermentation broth.

Hydrolysis of the BSG is performed for at least 12 hours, preferably 24 hours at a temperature in function of the enzyme(s) used (typically about 55°C), to ensure solubilization of arabinoxylans and increase in the level of WEAX to health-promoting levels of at least 1.4% (w/v). Hydrolysis is followed by a 8 to 24 hours of fermentation at about 25 to 37°C, preferably at 30°C. Preferably, the hydrolysis and fermentation steps are combined in one step (SSF) and performed during between 15 and 24h at a temperature between 25 and 37°C, more preferably during 20h at a temperature of 30°C. Aerobic and static conditions are used during the fermentation or SSF process.

The fermentation or SSF is followed by critical parameters such us pH, extract, total acidity (TTA) and concentration of reducing sugars. The process is considered to be finished when, for example, total acidity (TTA) doubles its value, preferably from 4.0 to 8.0 mL/10 mL of broth, and more preferably together with a drop of between 0.2 and 0.4 pH units and increased extract of 0.5-1.0% (extract measured by Anton-Paar and defined as gram of soluble solid per 100 g of broth). Alcohol concentration in the fermented broth is also measured. Aerobic and static conditions are used to ensure a low alcohol concentration, below 0.20%, preferably below 0.15%, and more preferable below 0.10% in the fermented broth.

At the end of fermentation, the fermentation broth typically has an acidity in a range of pH 3.5 - pH 4.5, preferably between pH 3.8 - pH 4.2, and more preferably between pH 3.9 - pH 4.1. The fermentation broth further is preferably low in fat content (<1.5%) and/or low in sugar content (<2.5%) and/or high in fiber content (>1.5 g fiber/ 100 kcal, preferably > 3 g fiber/ 100 kcal) and/or sufficient levels of health-promoting soluble arabinoxylans (no less than 1.4% w/v, preferably no less than 3%) and/or high in protein (>12%, preferably >20% of the energy provided by proteins) and/or very low in salt content (<0.4%).

Since no dairy product is used in the described process, the fermentation broth is consequently lactose free.

After fermentation, the pH of the fermentation broth is adjusted to a pH in a range of 2.5 to 3.5, preferably to a pH of 2.7 - preferably by additions of acids such as phosphoric acid and even more preferably by addition of strong acids such as sulfuric acid - allowing hydrolising the proteins in the fermentation broth by enzymatic treatment with eg. FP2 (Falcipain-2, a papain family cysteine protease).

Subsequently, proteinaceous material can be recovered (extracted, purified and/or separated) from the fermentation broth by for example an adsorption process. Such process may typically include three subsequent process steps. A first step in the protein recovery process is the separation of the solid particles. Typically, disc stack centrifuges, scroll decanters or hydrocyclones can be used for this purpose. A secondary solid removal step may be included to ensure that minimal quantities of particles are introduced to the equipment involved in subsequent protein purification steps. A failure to achieve this might imply a serious reduction in process outputs. Typical equipment used for secondary filtration might include filter bags or filter cartridges with a maximum pore diameter of 5, suitably 4, 3, 2 or 1 µm. The insoluble solids containing stream from protein recovery steps 1 and 2 above can be dried. For the recovery of proteinaceous material, the purified liquid stream comprising the hydrolyzed protein components is fed to a primary protein concentration process that can be achieved by a chromatography step. Types of chromatography that can be utilised include adsorption matrices with properties such as ion exchange (IEX), size exclusion, affinity or any other appropriate type used in liquid chromatography systems. After the primary protein concentration step, a further step might be necessary in order to increase concentration and the purity of a particular protein or proteins of interest. For this step an additional chromatographic step may be included. An ultrafiltration/diafiltration or evaporation step can be used to concentrate the protein mixture further after the chromatography steps. The type of filter for ultrafiltration/diafiltration will depend on the physical and chemical properties of the desired protein or proteins. A suitable filter material will then have for example, hydrophilic or hydrophobic properties and a nominal molecular weight cut off between 3-1000 kDa. A final step in the overall process includes further concentration, specifically removal of water.

Typical moisture content of protein powders are less than 20%. For this purpose, driers might be used that might include: cross-circulation and through-circulation driers, tray driers, tunnel driers, rotary driers, drum driers, spray driers and/or freeze drier. The protein depleted waste stream and optionally streams resulting from equilibration and regeneration of the adsorption matrix during used in the first or subsequent chromatography steps can enter waste water treatment systems. These streams are particularly suited to anaerobic digestion systems.

Examples of chromatography resins applicable in the first and further chromatography steps include, but are not limited to: Capto S (GE Healthcare) and Food-Grade Zeolite.

Elution of the proteinaceous material from the resins can be achieved by various eluents well known to persons skilled in the art and comprise, for example: NaCl solutions, NaHCO3 solutions, Na2CO3, NaOH, ...

The eluted proteinaceous material obtained by a method according to the present invention, is believed to have desired organoleptic properties and functionalities differing from proteinaceous material recovered from BSG without a step of fermenting the BSG. The proteinaceous material obtained by a method according to the present invention is believed to be particularly well suited for use as supplement (ingredient) for food and/or as foaming agent, emulsifying agent, egg or animal protein substitute in food recipes, diary protein substitute, baking ingredient, ...

Additionally, as the BSG derived product is maintained at an acidic pH, contamination of the BSG and the mircobiologycal spoilage is very limited if any at all.

### REFERENCES

Cao, L., Liu, X., Qian, T., Sun, G., Guo, Y., Chang, F., ... Sun, X. (2011). Antitumor and immunomodulatory activity of arabinoxylans: A major constituent of wheat bran. International Journal of Biological Macromolecules, 48(1), 160-164. doi:10.1016/j.ijbiomac.2010.10.014
Cloetens, L., Broekaert, W. F., Delaedt, Y., Ollevier, F., Courtin, C. M., Delcour, J. A., ... Verbeke, K. (2010). Tolerance of arabinoxylan-oligosaccharides and their prebiotic activity in healthy subjects: a randomised, placebo-controlled cross-over study. Br J Nutr, 103(5), 703-713. doi:10.1017/S0007114509992248
Efsa Panel on Dietetic Products, N. a. A. (2010). Scientific Opinion on Dietary Reference Values for carbohydrates and dietary fibre. EFSA Journal, 8(3), 1462-n/a. doi:10.2903/j.efsa.2010.1462
Efsa Panel on Dietetic Products, N. a. A. (2011). Scientific Opinion on the substantiation of health claims related to arabinoxylan produced from wheat endosperm and reduction of post-prandial glycaemic responses (ID 830) pursuant to Article 13(1) of Regulation (EC) No 1924/2006. EFSA Journal, 9(6), 2205-n/a. doi:10.2903/j.efsa.2011.2205
Efsa Panel on Dietetic Products, N. a. A. (2013). Scientific Opinion on Dietary Reference Values for energy. EFSA Journal, 11(1), 3005-n/a. doi:10.2903/j.efsa.2013.3005
Garcia, A. L., Otto, B., Reich, S. C., Weickert, M. O., Steiniger, J., Machowetz, A., ... Koebnick, C. (2006). Arabinoxylan consumption decreases postprandial serum glucose, serum insulin and plasma total ghrelin response in subjects with impaired glucose tolerance. European Journal of Clinical Nutrition, 61(3), 334. doi:10.1038/sj.ejcn.1602525
Lu, Z. X., Walker, K. Z., Muir, J. G., & Dea, K. O. (2004). Arabinoxylan fibre improves metabolic control in people with Type II diabetes. European Journal of Clinical Nutrition, 58(4), 621. doi:10.1038/sj.ejcn.1601857
Reis, S. F., Abu-Ghannam, N., Gullón, B., Gullón, P., Ferreira, S., Maia, C. J., ... Alonso, J. L. (2014). Evaluation of the prebiotic potential of arabinoxylans from brewer's spent grain. Applied Microbiology and Biotechnology, 98(22), 9365-9373. doi:10.1007/s00253-014-6009-8
Sanchez, J. !., Marzorati, M., Grootaert, C., Baran, M., Verstraete, V., Van De Wiele, C. M., .. . Delcour, T. (2009). Arabinoxylan-oligosaccharides (AXOS) affect the protein/carbohydrate fermentation balance and microbial population dynamics of the Simulator of Human Intestinal Microbial Ecosystem. Microbial Biotechnology, 2(1), 101-113. doi:10.1111/j.1751-7915.2008.00064.x
Teixeira, C., Nyman, M., Andersson, R., & Alminger, M. (2017). Application of a dynamic gastrointestinal in vitro model combined with a rat model to predict the digestive fate of barley dietary fibre and evaluate potential impact on hindgut fermentation. Bioactive Carbohydrates and Dietary Fibre, 9, 7-13. doi:10.1016/j.bcdf.2016.12.001
Zhou, S., Liu, X., Guo, Y., Wang, Q., Peng, D., & Cao, L. (2010). Comparison of the immunological activities of arabinoxylans from wheat bran with alkali and xylanase-aided extraction. Carbohydrate Polymers, 81(4), 784-789. doi:10.1016/j.carbpol.2010.03.040

## Claims

1. A process of extracting or purifying proteinaceous material and fibraceous material from brewer's spent grain (BSG), the process comprising the steps of:
a) Providing brewer's spent grain;
b) Performing enzymatic treatment of the brewer's spent grain, the enzymatic treatment of the brewer's spent grain including addition of xylanase, to form an enzymatically treated brewer's spent grain;
c) Fermenting the enzymatically treated brewer's spent grain with lactic acid bacteria and/or acetic acid bacteria and/or probiotics to obtain a fermented broth;
d) Hydrolysing proteins in the fermented broth to provide a hydrolysed fermented broth, wherein the pH of the fermentation broth is adjusted to a pH in the range of 2.5 to 3.5 for the step of hydrolysing; and,
e) Extracting and/or purifying proteinaceous and fibraceous material from the hydrolysed fermented broth.

2. The process according to claim 1, wherein brewer's spent grain is treated with enzymes to solubilize arabinoxylans.

3. The process according to any of the preceding claims, the enzyme treatment of the brewer's spent grain further including addition of one or more enzymes with following enzymatic activity to the brewer's spent grain: alpha-amylase, gluco-amylase, cellulase, protease, beta-glucanase and/or admixtures thereof.

4. The process according to any of the preceding claims, wherein the pH of the fermentation broth at the end of fermentation ranges between pH 3.5 - pH 4.5, preferably between pH 3.8 - pH 4.2, and more preferably between pH 3.9 - pH 4.1.

5. The process according to any of the preceding claims, wherein the pH of the fermentation broth is adjusted to a pH of 2.7 for the step of hydrolysing proteins.

6. The process according to claim 5, wherein the pH of the fermentation broth is adjusted by addition of sulfuric acid and/or phosphoric acid.

7. Use of proteinaceous material obtained from a process as identified in any of claims 1-6 as a food supplement.

8. Use of proteinaceous material obtained from a process as identified in any of claims 1-6 as a feed supplement.

9. Use of proteinaceous material obtained from a process as identified in any of claims 1-6 as foaming agent, emulsifying agent, animal or egg protein substitute, dairy protein substitute and/or baking ingredient.

10. Use of a lactic acid bacteria, preferably of the species *Lactobacillus plantarum* and/or *Lactobacillus rhamnosus,* more preferably the strain *Lactobacillus plantarum* F10 and/or *Lactobacillus rhamnosus* GG (LGG^{®}), in the process according to any one of claims 1-6 for recovering proteinaceous or fibrous material from BSG.

## Patentansprüche

1. Verfahren zum Extrahieren oder Aufreinigen von Protein- und Fasermaterial aus Brauereitreber (BSG), wobei das Verfahren die folgenden Schritte umfasst:
a) Bereitstellen von Brauereitreber;
b) Durchführen einer enzymatischen Behandlung des Brauereitrebers, wobei die enzymatische Behandlung des Brauereitrebers den Zusatz von Xylanase einschließt, um einen enzymatisch behandelten Brauereitreber zu bilden;
c) Fermentieren des enzymatisch behandelten Brauereitrebers mit Milchsäurebakterien und/oder Essigsäurebakterien und/oder Probiotika, um eine fermentierte Brühe zu erzielen;
d) Hydrolysieren von Proteinen in der fermentierten Brühe, um eine hydrolysierte fermentierte Brühe bereitzustellen, wobei der pH-Wert der Fermentationsbrühe für den Schritt der Hydrolyse auf einen pH-Wert im Bereich von 2,5 bis 3,5 eingestellt wird; und,
e) Extrahieren und/oder Aufreinigen von Protein- und Fasermaterial aus der hydrolysierten fermentierten Brühe.

2. Verfahren nach Anspruch 1, wobei Brauereitreber mit Enzymen zur Solubilisierung von Arabinoxylanen behandelt wird.

3. Verfahren nach einem der vorhergehenden Ansprüche, wobei die Enzymbehandlung des Brauereitrebers ferner die Zugabe eines oder mehrerer Enzyme mit folgender enzymatischer Aktivität zum Brauereitreber einschließt: Alpha-Amylase, Gluco-Amylase, Cellulase, Protease, Beta-Glucanase und/oder Gemische davon.

4. Verfahren nach einem der vorhergehenden Ansprüche, wobei der pH-Wert der Fermentationsbrühe am Ende der Fermentation zwischen pH 3,5 - pH 4,5, vorzugsweise zwischen pH 3,8 - pH 4,2 und noch bevorzugter zwischen pH 3,9 - pH 4,1 liegt.

5. Verfahren nach einem der vorhergehenden Ansprüche, wobei der pH-Wert der Fermentationsbrühe für den Schritt der Hydrolyse von Proteinen auf einen pH-Wert von 2,7 eingestellt wird.

6. Verfahren nach Anspruch 5, wobei der pH-Wert der Fermentationsbrühe durch Zugabe von Schwefelsäure und/oder Phosphorsäure eingestellt wird.

7. Verwendung von Proteinmaterial, erzielt durch ein wie in einem der Ansprüche 1 bis 6 identifiziertes Verfahren, als Nahrungsergänzungsmittel

8. Verwendung von Proteinmaterial, erzielt durch ein wie in einem der Ansprüche 1 bis 6 identifiziertes Verfahren, als Nahrungsergänzungsmittel.

9. Verwendung von Proteinmaterial, erzielt durch ein wie in einem der Ansprüche 1 bis 6 identifiziertes Verfahren, als Schaummittel, Emulgator, Ersatz für tierisches oder Eiprotein, Ersatz für Milchprotein und/oder Backzutat.

10. Verwendung eines Milchsäurebakteriums, vorzugsweise der Spezies *Lactobacillus plantarum* und/oder *Lactobacillus rhamnosus,* besonders bevorzugt des Stammes *Lactobacillus plantarum* F10 und/oder *Lactobacillus rhamnosus* GG (LGG^{®}), in dem Verfahren nach einem der Ansprüche 1 bis 6 zur Gewinnung von Protein- oder Fasermaterial aus BSG.

## Revendications

1. Procédé d'extraction ou de purification de matières protéinées et de matières fibreuses à partir de drêches de brasserie (BSG), le procédé comprenant les étapes consistant à :
a) fournir les drêches de brasserie ;
b) réaliser un traitement enzymatique des drêches de brasserie, le traitement enzymatique des drêches de brasserie incluant l'ajout de xylanase, pour former des drêches de brasserie traitées de manière enzymatique ;
c) faire fermenter les drêches de brasserie traitées de manière enzymatique avec des bactéries d'acide lactique et/ou des bactéries d'acide acétique et/ou des probiotiques pour obtenir un bouillon fermenté ;
d) hydrolyser des protéines dans le bouillon fermenté pour fournir un bouillon fermenté hydrolysé, dans lequel le pH du bouillon de fermentation est ajusté à un pH dans la plage de 2,5 à 3,5 pour l'étape d'hydrolyse ; et
e) extraire et/ou purifier des matières protéinées et fibreuses à partir du bouillon fermenté hydrolysé.

2. Procédé selon la revendication 1, dans lequel les drêches de brasserie sont traitées avec des enzymes pour solubiliser des arabinoxylanes.

3. Procédé selon la revendication quelconque des revendications précédentes, le traitement enzymatique des drêches de brasserie incluant en outre l'ajout d'une ou plusieurs enzymes avec une activité enzymatique suivante aux drêches de brasserie : alpha-amylase, gluco-amylase, cellulase, protéase, bêta-glucanase et/ou des mélanges de celles-ci.

4. Procédé selon l'une quelconque des revendications précédentes, dans lequel le pH du bouillon de fermentation à la fin de la fermentation est compris entre un pH de 3,5 et un pH de 4,5, de préférence entre un pH de 3,8 et un pH de 4,2, et de manière davantage préférée entre un pH de 3,9 et un pH de 4,1.

5. Procédé selon l'une quelconque des revendications précédentes, dans lequel le pH du bouillon de fermentation est ajusté à un pH de 2,7 pour l'étape d'hydrolyse de protéines.

6. Procédé selon la revendication 5, dans lequel le pH du bouillon de fermentation est ajusté par addition d'acide sulfurique et/ou d'acide phosphorique.

7. Utilisation d'une matière protéinée obtenue à partir d'un procédé tel qu'identifié dans l'une quelconque des revendications 1 à 6 comme complément nutritionnel.

8. Utilisation d'une matière protéinée obtenue à partir d'un procédé tel qu'identifié dans l'une quelconque des revendications 1 à 6 comme complément alimentaire.

9. Utilisation d'une matière protéinée obtenue à partir d'un procédé tel qu'identifié dans l'une quelconque des revendications 1 à 6 en tant qu'agent moussant, agent émulsionnant, substitut de protéine animale ou d'oeuf, substitut de protéine du lait et/ou ingrédient de boulangerie.

10. Utilisation d'une bactérie d'acide lactique, de préférence de l'espèce *Lactobacillus plantarum* et/ou *Lactobacillus rhamnosus,* de préférence de la souche *Lactobacillus plantarum* F10 et/ou *Lactobacillus rhamnosus* GG (LGG^{®}), dans le procédé selon l'une quelconque des revendications 1 à 6, pour récupérer de la matière protéinée ou fibreuse à partir de BSG.
